Europäisches Patentamt

European Patent Office

Office européen des brevets

(11) Publication number: **0 092 999 B1**

# EUROPEAN PATENT SPECIFICATION

(45) Date of publication of patent specification: **01.04.92**

(51) Int. Cl.5: **A61L 15/16**, A61K 31/715, A61K 31/725, A61K 37/12

(21) Application number: **83302316.1**

(22) Date of filing: **22.04.83**

Divisional application 88201241 filed on 20.06.88.

The file contains technical information submitted after the application was filed and not included in this specification

(54) **Dressing.**

(30) Priority: **22.04.82 US 370893**

(43) Date of publication of application:
**02.11.83 Bulletin 83/44**

(45) Publication of the grant of the patent:
**01.04.92 Bulletin 92/14**

(84) Designated Contracting States:
**AT BE CH DE FR GB IT LI LU NL SE**

(56) References cited:
EP-A- 0 017 401       EP-A- 0 018 093
EP-A- 0 026 572       EP-A- 0 063 898
EP-A- 0 081 907       FR-A- 2 392 076
FR-A- 2 393 566       GB-A- 2 000 683
GB-A- 2 061 732       US-A- 3 972 328
US-A- 4 192 785       US-A- 4 225 580
US-A- 4 292 972

CHEMICAL ABSTRACTS, vol. 98, no. 9, Febr. 28, 1983, page 28, ref. no. 65221r, Columbus, Ohio, US; G.FURNADZHIEV et al.: "Experimental study of granulated pectin with a moderate degree of esterification for antiinflammatory activity"

(73) Proprietor: **E.R. Squibb & Sons, Inc.**
**Lawrenceville-Princeton Road**
**Princeton, N.J. 08540-4000(US)**

(72) Inventor: **Pawelchak, John M.**
**J-20 Avon Drive**
**East Windsor New Jersey(US)**
Inventor: **Freeman, Frank M.**
**10 Brandon Road**
**Lawrenceville New Jersey(US)**

(74) Representative: **Thomas, Roger Tamlyn et al**
**D. Young & Co. 10 Staple Inn**
**London WC1V 7RD(GB)**

## Description

This invention is directed to an occlusive dressing useful for treating skin lesions such as dermal ulcers and pressure sores.

US Patent US-A-3,972,328 describes a medical dressing or bandage which comprises (i) an adhesive layer comprising a pressure-sensitive rubbery elastomer adhesive material such as a mixture of polyisobutylenes of a molecular weight of 10,000 to 11,700 and 81,000 to 99,000 having intimately dispersed therein a water-soluble or swellable hydrocolloid or a mixture of hydrocolloids such as gelatin and sodium carboxymethylcellulose, a tackifier such as terpene resin and a plasticiser or solvent such as mineral oil, (ii) a semi-open cell polymeric foam layer and (iii) an outer polymeric film coating. In the Example, (i) contains a high proportion of terpene resin and mineral oil.

French Patent Application FR-A-2393566 describes a hand-mouldable composition primarily for adhering an ostomy device to the skin. The composition comprises a pressure-sensitive adhesive material such as a lower molecular weight polyisobutylene, and a hydrocolloid material. Similarly GB Patent Application GB-A-2000683 describes an ostomy appliance having a faceplate to which is applied a homogeneous mixture of gelatin, pectin, sodium carboxymethylcellulose and polyisobutylene as an adhesive.

According to the present invention, there is provided an occlusive multi-layered dressing comprising an adhesive layer which, in use, contacts the wound and surrounding normal skin, an intermediate layer, of semi-open-cell polymeric foam, and an outer moisture impervious polymeric film coated or laminated to the upper surface of said foam layer wherein said wound and skin contacting adhesive layer consists essentially of a homogeneous blend of from about 35% to about 50% by weight of one or more low molecular weight polyisobutylenes which act as pressure sensitive adhesive materials and from about 45% to about 65% by weight of one or more water dispersable hydrocolloids selected from sodium carboxymethylcellulose, calcium carboxymethylcellulose, pectin, gelatin, guar gum, locust bean gum, collagen, and gum karaya.

Embodiments of the invention will now be described by way of example with reference to the drawings in which:-

Fig. 1 is an overall view of one embodiment of the composite dressing of this invention (greatly enlarged).

Fig. 2 is a front view along line 2-2 of Fig. 1.

Fig. 3 is an overall view of another embodiment of the composite dressing of this invention (greatly enlarged).

Fig. 4 is a front view along line 3-3 of Fig. 3.

Fig. 5 is an overall view of another embodiment of the composite dressing of this invention (greatly enlarged).

Fig. 6 is a front view along line 5-5 of Fig. 5.

Fig. 7 is a top view of an open wound covered by the dressing and partially cut-away to show the granular packing.

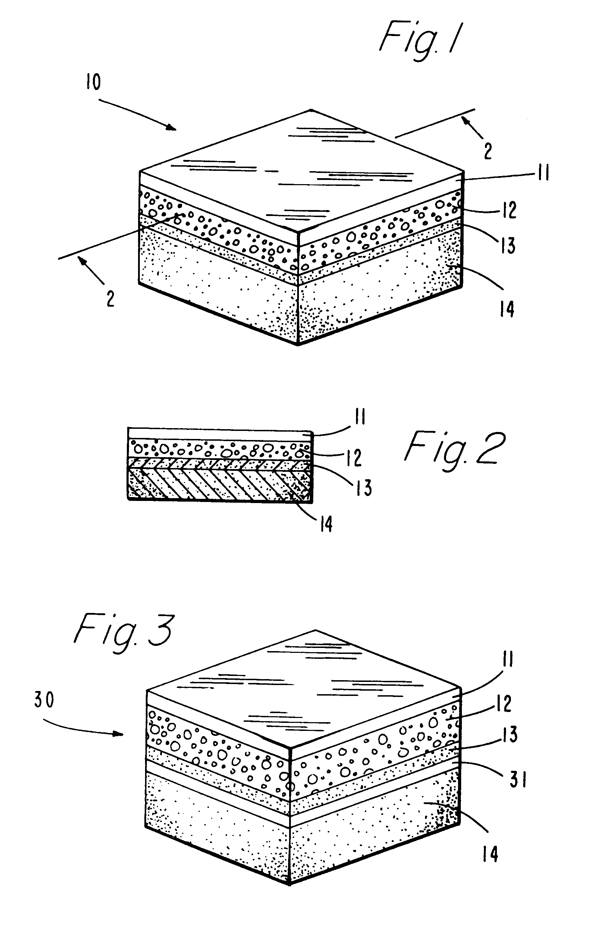

One embodiment of the occlusive dressing 10 of this invention as shown in figs 1 and 2 comprises a first adhesive layer 14 which is formulated from materials selected to interact with fluid discharged from the wound as well as forming a fluid-tight bond with the healthy skin around the wound so as to seal the dressing to the skin. The dressing of this embodiment includes a second adhesive layer 13 formulated of materials which can be cast onto foam layer 12 and will form an aggressive bond when pressed into contact with adhesive layer 14. The layer 12 is a semi-open cell elastic or flexible foam. The outer layer 11 can be a polymeric film or a skin formed on the top of foam layer 12 which serves to protect the exposed surface of the dressing from contamination by water or soil.

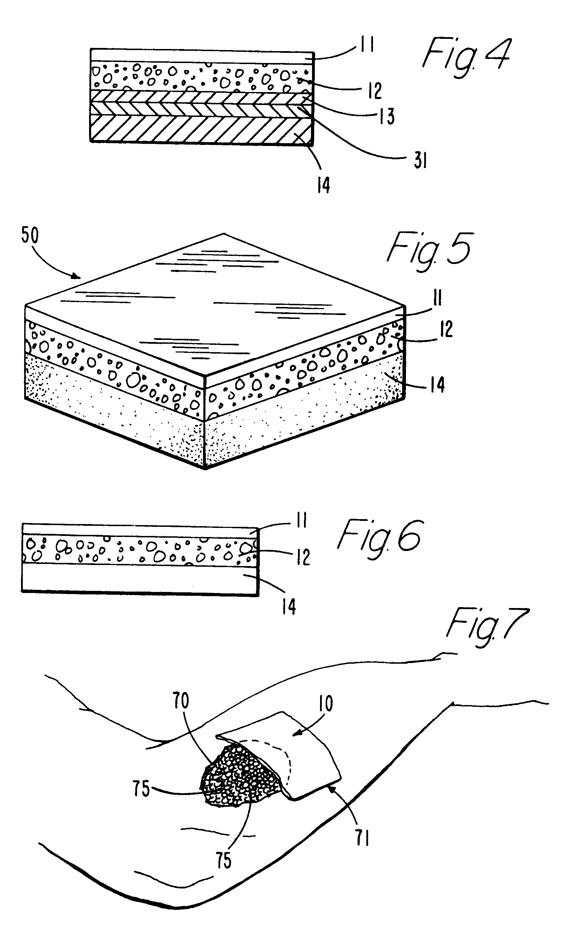

An alternative embodiment of the occlusive dressing is shown as 30 in figs. 3 and 4. Dressing 30 includes a layer of deodorizing material designated 31 interposed between adhesive layers 14 and 13.

Another alternative embodiment of the occlusive dressing is shown as 50 in figs. 5 and 6. Dressing 50 omits second adhesive layer 13 and has adhesive layer 14 laminated directly to the semi-open cell elastic foam 12.

Adhesive layer 14 comprises a homogeneous blend of one or more pressure sensitive adhesive materials which are low molecular weight polyisobutylenes, and one or more water dispersable hydrocolloidal materials of the group defined above, in the percentages defined above. Adhesive layer 13 comprises a homogeneous blend of one or more pressure sensitive adhesive materials, one or more water dispersable hydrocolloidal materials, a tackifier, and a plasticizer or solvent. Additionally, one or more thermoplastic elastomers may be included with the pressure sensitive adhesive materials in layer 13 and one or more water swellable cohesive strengthening agents and/or one or more natural or synthetic polymers capable of developing elastomeric properties when hydrated may be included with the hydrocolloidal materials in layer 13.

Suitable pressure sensitive adhesive materials for inclusion in layer 13 are various natural or synthetic viscous or elastomeric substances such as natural rubber, silicone rubber, acrylonitrile rubber, polyurethane rubber, polyisobutylene, etc. Low molecular weight polyisobutylenes having a viscosity average molecular weight of from about 36,000 to about 58,000 (Florey) possessing pressure sensitive adhesive properties are preferred. Such polyisobutylenes are commercially available under the trademark Vistanex from Exxon as grades LM-MS and LM-MH and may also be used for layer 14.

Optionally, one or more thermoplastic elastomers can be included in the pressure sensitive adhesive component of layer 13. These elastomers impart the properties of rubber-like extensibility and both rapid and complete recovery from modular strains to the pressure sensitive adhesive component. Suitable thermoplastic elastomers include medium molecular weight polyisobutylenes having a viscosity average molecular weight of from about 1,150,000 to 1,600,000 (Florey), butyl rubber which is a copolymer of isobutylene with a minor amount of isoprene having a viscosity average molecular weight of from about 300,000 to about 450,000 (Florey), and styrene copolymers such as styrene-butadienestyrene (S-B-S), styrene-isoprene-styrene (S-I-S), and styrene-ethylene/butylene-styrene (S-EB-S) which are commercially available, for example, from Shell Chemical Co. under the trademark Kraton as Kraton D1100, D1102, Kraton D1107, Kraton 4000, Kraton G1600, and Kraton G4600. Preferred thermoplastic elastomers are butyl rubber having a viscosity average molecular weight of about 425,000 (commercially available from Exxon as grade 077), polyisobutylene having a viscosity average molecular weight of about 1,200,000 (commercially available under the trademark Vistanex from Exxon as grade L-100), and styrene-isoprene-styrene (S-I-S)-copolymers (commercially available from Shell as Kraton D1107).

The pressure sensitive adhesive component including the optional thermoplastic elastomer is present in adhesive layer 13 at from about 30% to about 70% by weight of the composition, preferably from about 35% to about 50% by weight. The thermoplastic elastomer can be employed at up to three times the weight of the pressure sensitive elastomeric substances but preferably the theromoplastic elastomer if present will be at from about 20% to about 150% by weight of the pressure sensitive elastomeric substance.

Adhesive layer 13 includes from about 10% to about 65% by weight of one or more water dispersable hydrocolloid materials. Suitable hydrocolloid materials include sodium or calcium carboxymethylcellulose, pectin, gelatin, guar gum, locust bean gum, collagen and gum karaya. Adhesive layer 13 may also include up to about 50% by weight of one or more water swellable cohesive strengthening agents and/or one or more natural or synthetic polymers capable of developing elastomeric properties when hydrated provided that the water soluble hydrocolloid gums, water swellable cohesive strengthening agents, and hydratable polymers together are present at no more than about 70% by weight of adhesive layer 13. Suitable water swellable cohesive strengthening agents include finely divided substantially water insoluble cross-linked sodium carboxymethylcellulose such as that commercially available under the trademark Aqualon or that described in U.S. Patent 3,589,364 and available commercially from the Buckeye Cellulose Corp., finely divided substantially water insoluble starch-acrylonitrile graft copolymer such as that described in U.S. Patent 3,661,815 and commercially available from the Grain Processing Corp., and finely divided substantially water insoluble cross-linked dextran such as that commercially available under the trademark Sephadex. Suitably hydratable polymers are gluten and long chain polymers of methyl vinyl ether/maleic acid, preferably, the long chain polymers of methyl vinyl ether/maleic acid commercially available under the trademark Gantrez from GAF Inc. The maleic acid moiety in the polymer may be intact (Gantrez S-97), may be an anhydride (Gantrez AN-169), or may be a metal salt such as the mixed sodium/calcium salts (Gantrez AT-955).

Preferably, the water dispersable hydrocolloids, the optional water swellable cohesive strengthening agents, and the optional hydratable polymers are present at from about 45% to about 65% by weight of adhesive layer 14 and from about 30% to about 50% by weight of adhesive layer 13.

The water dispersable hydrocolloids provide wet tack for adhesive layers 14 and 13 while the pressure sensitive adhesive component provides dry adhesion and imparts structural integrity to layers 14 and 13.

Adhesive layer 13 also includes from about 5% to about 15% by weight of a plasticizer or solvent such as mineral oil or petrolatum with mineral oil being preferred and from about 15% to about 25% by weight of a tackifier such as a terpene resin.

Small amounts, i.e., less than 5% by weight, of other ingredients may be included within adhesive layers 14 and 13. For example, an antioxidant such as butylated hydroxyanisole or butylated hydroxytoluene, a deodorant such as chlorophyllins, or a perfume agent may be included. In addition, small amounts of a pharmacologically active ingredient can be included in adhesive layer 14. For example, an antibiotic or antimicrobial agent such as neomycin, an antiseptic agent such as povidone iodine, and an antiinflammatory agent such as hydrocortisone or triamcinolone acetonide.

The semi-open cell elastic or flexible foam layer 12 can be formed from various elastomer materials such as polyester or polyether polyurethane foams, styrene-butadiene foams, certain rubber based foam, etc. The material should, of course, be non-toxic and stable. The preferred material is a flexible polyurethane foam having from about 50 to about 100 cells per linear inch (2.54 cm) with about 80 cells per linear inch (2.54 cm) being most preferred. By semi-open it is meant that the percentage of open or ruptured cells is from about 30 to about 85%.

The outer layer 11 can be a polymeric elastic or flexible film coating formed from a water impermeable pliable elastomer material such as flexible polyurethane, polyacrylate, polyethylene,etc. Layer 11 can be a skin of such polymeric material flame laminated to the top of foam layer 12 by means of heat and/or pressure. The exposed sides of polymeric foam layer 12 can also be coated or heat and/or pressure treated to form an impermeable film or skin. Polyurethane is the preferred material for film or skin 11.

In a typical dressing, adhesive layer 14 will vary in thickness from about 0.02 inches (0.5 mm) to about 0.1 inches (2.54 mm), preferably about 0.05 inches (1.27 mm), adhesive layer 13 will vary in thickness from about 0.005 inches (0.127 mm) to about 0.02 inches (0.5 mm), preferably about 0.015 inches (0.38 mm), foam layer 12 will vary in thickness from about 0.03 inches (0.76 mm) to about 0.1 inches (2.54 mm), preferably about 0.065 inches (1.65 mm) and outer layer or skin 11 will vary in thickness from about 0.001 inches (0.0254 mm) to about 0.003 inches (0.076 mm), preferably about 0.002 inches (0.05 mm).

As shown in figs. 3 and 4, a layer of deodorizing material can be included between the two adhesive layers. Suitable deodorizing materials include a sheet of foamed polymeric material such as polyurethane having a large number of activated carbon particles bound to the matrix of the foam. Such a material is commercially available under the tradename Bondina. Another type of deodorizing material is a paper or felt like substance containing activated carbon such as that commercially available under the tradename K-felt (Toyobo) or Getter paper (Mead). The layer of deodorizing material will vary from about .010 to 0.100 inches (0.254 to 2.54 mm).

The dressing 10 is prepared as follows. First, adhesive layer 14 is prepared by forming a homogeneous dispersion of the pressure sensitive adhesive material with a heavy duty mixer, e.g., a kneader mixer or sigma blade mixer. The hydrocolloid gums, water swellable cohesive strengthening agents, hydratable polymers, and any other optional ingredients are added and mixing is continued until a homogeneous dough is formed. This dough is then extruded into a thick slab which is thinned down by pressure rollers to the desired thickness.

Next, adhesive layer 13 is prepared by forming a mixture of the hydrocolloid gums, pressure sensitive adhesive materials, tackifier and plasticizer, as well as other optional ingredients such as thermoplastic elastomers, water swellable cohesive strengthening agents, hydratable polymers, antioxidants, etc., in an organic solvent such as heptane or hexane. The resulting adhesive slurry is then applied to a web of silicone coated release paper and the solvent is evaporated. Upon drying the hydrocolloids are dispersed throughout the adhesive layer. This adhesive material is then compressed with a laminate of semi-open cell flexible polymeric foam having a water impermeable polymeric coating or skin on one side.

Finally, the silicone coated release paper is stripped away from adhesive layer 13 and adhesive layers 13 and 14 are pressed together with heat to form the dressing. Silicone coated release paper can then be applied to the exposed surface of adhesive layer 14 and the dressing can be cut to the desired shape and packaged. After packaging, the dressing can be sterilized by gamma irradiation.

Dressing 30 is prepared in a similar manner except that a layer of deodorizing material is laminated between adhesive layers 14 and 13. Alternatively, dressing 30 can be prepared by first laminating adhesive layer 13 directly to the layer of deodorizing material 31 and then attaching this adhesive coated material to foam layer 12. In the case of dressing 50, adhesive layer 14 is prepared as described above and is then laminated while warm directly to foam layer 12 by means of pressure.

The following are representative examples of dressings within the scope of the invention.

Example 1

A dressing was prepared having the following composition

Layers 11 and 12

Semi-open cell polyurethane foam having a polyurethane skin flame laminated to one surface.

4

| Layer 13 | |
|---|---|
| | Percent by weight |
| Polyisobutylene (Vistanex LM-MH) | 18.0 |
| Polyisobutylene (Vistanex L-100) | 20.0 |
| Terpene resin (Piccolyte) | 20.0 |
| Butylated hydroxytoluene | 0.5 |
| Mineral oil | 8.5 |
| Sodium carboxymethylcellulose | 18.0 |
| Gelatin | 15.0 |

| Layer 14 | |
|---|---|
| | Percent by weight |
| Polyisobutylene (Vistanex LM-MH) | 40 |
| Gelatin | 20 |
| Pectin | 20 |
| Sodium carboxymethylcellulose | 20 |

Adhesive layer 14 was prepared as follows. A premix was prepared by blending 1.4 kg. of gelatin, 1.4 kg. of pectin, and 1.4 kg. of sodium carboxymethylcellulose. The blended premix was added to a heavy duty sigma blade-type mixer followed by the addition of 1.4 kg. of polyisobutylene. After mixing for 10 minutes, an additional 1.4 kg. of polyisobutylene was added and mixing continued until a homogeneous dough was formed (about 10 to 20 minutes). This dough mass while hot and soft was extruded and thinned down by pressure rollers to a thickness of about 0.05 inches (1.27 mm).

A laminate of adhesive layer 13 to semi-open cell polymeric foam (layers 12 and 11) was prepared as follows.

A mixture of 0.31 kg. of polyisobutylene (Vistanex L-100), 0.28 kg. of sodium carboxymethylcellulose, and 0.23 kg. of gelatin was kneaded. This mixture was added to a solution of heptane containing 0.28 kg. of polyisobutylene (Vistanex LM-MH), 0.31 kg. of piccolyte resin, 8 g. of butylated hydroxytoluene, and 0.13 kg. of mineral oil to form an adhesive slurry. A portion of this slurry was poured onto a sheet of silicone coated release paper and the solvent evaporated to leave an adhesive layer of about 0.015 inches (0.38 mm) in thickness . This adhesive layer was laminated to a semi-open cell polyurethane foam of 0.065 inches (1.65 mm) thickness having a polyurethane skin of about 0.002 inches (0.05 mm) thickness on one surface by gently compressing the adhesive layer to the foam by passing through pressure rollers.

The silicone coated release paper was then stripped from adhesive layer 13 and adhesive layer 13 and adhesive layer 14 were compressed together by passing through pressure rollers. Silicone coated release paper was then pressed onto the exposed surface of adhesive layer 14. The resulting dressing was cut into the desired shape and packaged.

Example 2

Following the procedure of Example 1 but employing 40% Polyisobutylene (Vistamex LM-MH) and 60% Guar gum on a weight percent basis in adhesive layer 14 another dressing within the scope of this invention is obtained.

Examples 3 - 10

Following the procedure of Example 1 but employing the ingredients listed below on a weight percent basis in adhesive layer 13 other dressings within the scope of this invention are obtained.

| Ingredient | Example | | | |
| --- | --- | --- | --- | --- |
| | 3 | 4 | 5 | 6 |
| Polyisobutylene (Vistanex LM-MH) | 15 | 15 | 15 | 15 |
| Guar gum | 20 | 15 | - | - |
| Locust bean gum | - | - | 17 | - |
| Pectin | - | - | - | 10 |
| Karaya | - | - | - | - |
| Gelatin | - | - | - | 10 |
| Sodium carboxymethylcellulose | 15 | 15 | 10 | 10 |
| Collagen | - | - | - | - |
| Cross-linked sodium carboxymethylcellulose (Aqualon R) | - | 15 | - | - |
| Starch-acrylonitrile graft copolymer (Grain Processing Corp. Polymer 35-A-100) | - | - | - | - |
| Cross-linked dextran (Sephadex CM-C50) | - | - | - | 10 |
| Poly(methyl vinyl ether/maleic acid), mixed calcium, sodium salt (Gantrez AT-955) | - | - | 10 | - |
| Polyisobutylene (Vistanex L-100) | - | - | 20 | 15 |
| Butyl rubber (grade 077) | 20 | - | - | - |
| Styrene-isoprene copolymer (Kraton 1107) | - | 19.5 | - | - |
| Mineral oil | 9.5 | 5 | 7.5 | 9.5 |
| Picolyte resin | 20 | 15 | 20 | 20 |
| Butylated hydroxytoluene | 0.5 | 0.5 | 0.5 | 0.5 |

| Ingredient | Example | | | |
| --- | --- | --- | --- | --- |
| | 7 | 8 | 9 | 10 |
| Polyisobutylene (Vistanex LM-MH) | 15 | 15 | 20 | 15 |
| Guar gum | 15 | 15 | 25 | - |
| Locust bean gum | - | - | - | - |
| Pectin | - | - | - | - |
| Karaya | - | - | - | 10 |
| Gelatin | - | - | - | - |
| Sodium carboxymethylcellulose | 10 | - | - | 15 |
| Collagen | - | - | 5 | - |
| Cross-linked sodium carboxymethylcellulose (Aqualon R) | - | 10 | - | - |
| Starch-acrylonitrile graft copolymer (Grain Processing Corp. Polymer 35-A-100) | 10 | - | - | - |
| Cross-linked dextran (Sephadex CM-C50 | - | - | - | 10 |
| Poly(methyl vinyl ether/maleic acid), mixed calcium, sodium salt (Gantrez AT-955) | - | 10 | - | - |
| Polyisobutylene (Vistanex L-100) | 20 | 20 | 15 | 20 |
| Butyl rubber (grade 077) | - | - | - | - |
| Styrene-isoprene copolymer (Kraton 1107) | - | - | - | - |
| Mineral oil | 9.5 | 9.5 | 9.5 | 9.5 |
| Picolyte resin | 20 | 20 | 25 | 20 |
| Butylated hydroxytoluene | 0.5 | 0.5 | 0.5 | 0.5 |

The wound packing material is a granular product of from about 10 to about 40 mesh particle size and comprises a water dispersable hydrocolloidal material or a mixture of such materials. The granular product can also optionally include up to about 50% by weight of one or more water swellable cohesive strengthening agents and/or one or more hydratable polymers. Suitable water dispersable hydrocolloidal materials include sodium and calcium carboxymethylcellulose, pectin, gelatin, guar gum, locust bean gum, collagen, and gum karaya. Suitable water swellable cohesive strengthening agents include finely divided substantially water insoluble cross-linked sodium carboxymethylcellulose such as that commercially available under the trademark Aqualon or that described in U.S. Patent 3,589,364 and available commercially from the Buckeye Cellulose Corp., finely divided substantially water insoluble starch-acrylonitrile graft copolymer such as that described in U.S. Patent 3,661,815 and commercially available from the Grain Processing Corp., and finely divided substantially water insoluble cross-linked dextran such as that

6

commercially available under the trademark Sephadex. Suitably hydratable polymers are gluten and long chain polymers of methyl vinyl ether/maleic acid, preferably, the long chain polymers of methyl vinyl ether/maleic acid commercially available under the trademark Gantrez from GAF Inc. The maleic acid moiety in the polymer may be intact (Gantrez S-97), may be an anhydride (Gantrez AM-169), or may be a metal salt such as the mixed sodium/calcium salts (Gantrez AT-955).

Small amounts, i.e., less than 5% by weight, of other ingredients may be included within or sprayed onto the granules. For example, an antioxidant such as butylated hydroxyanisole or butylated hydroxytoluene, a deodorant such as chlorophyllins, or a perfume agent may be included. In addition, small amounts of a pharmacologically active ingredient can be included within or sprayed onto the granules. For example, an antibiotic or antimicrobial agent such as neomycin, an antiseptic agent such as povidone iodine, and an antiinflammatory agent such as hydrocortisone or triamcinolone acetonide.

The granules preferably contain at least 70% by weight of one or more water dispersable hydrocolloids selected from pectin, gelatin, sodium carboxymethylcellulose, and collagen with a granular product consisting of an equal weight percent mixture of pectin, gelatin, and sodium carboxymethylcellulose being most preferred.

The granular material can be prepared from the powder ingredients by dry compaction, wet granulation, or fluidized bed granulation techniques. The dry compaction method involves blending the component powders, compacting into a slab, and milling the slab to the desired particle size. The milled material is sieved and particles of the proper size range are gathered and packaged.

In the wet granulation process, the powders after blending, or simultaneously with blending, are moistened with water, a hydroalcoholic solution, or low concentration dispersion of one or more of the water dispersable hydrocolloids in water or hydroalcoholic vehicles. Normally, the amount of moisture added is up to about 50% of the dry weight of the powders in order to form granules with adequate process-ability and resistance to attrition. After thorough mixing, the moistened mass is forced through a screen or die to yield granules directly or to yield by extrusion, a particulate noodle-like or ribbon-like mass of material. When dried, this material is then milled to the desired sieve size and packaged. Alternatively, the moistened mass may first be broken into large lumps which are then dried and milled to the desired particle size.

In the fluid bed granulation procedure, the moistening fluid is added to a charge of the blended powders held suspended on a column of rising warm air where the powders are allowed to mix with the granulating fluid, which is very rapidly evaporated, leaving behind agglomerated granules. Less moistening fluid is required in this process then in the wet granulation process described above.

Example 11

To 120 kg. of a mixture of equal parts of gelatin, pectin and sodium carboxymethylcellulose were added about 50 liters of distilled water or a mixture of water and ethanol. Mixing was performed in a rotating oscillating device to produce a homogeneous blend. The blend was screened through a coarse sieve (0.5 inch = 12.7 mm) and the moistened material was dried at 55°C for 24 hours. The dried material was milled and screened to between 16 and 20 mesh.

Examples 12 - 22

Following the procedure of Example 11 but employing the ingredients listed below on a weight percent basis other granular products within the scope of this invention are obtained.

7

| Ingredient | Example | | | |
|---|---|---|---|---|
| | 12 | 13 | 14 | 15 |
| Guar gum | - | - | - | - |
| Locust bean gum | - | - | - | - |
| Pectin | 25 | 50 | - | - |
| Karaya | - | - | - | - |
| Gelatin | 25 | - | 50 | - |
| Sodium carboxymethylcellulose | 25 | - | - | 50 |
| Calcium carboxymethylcellulose | - | - | - | - |
| Collagen | 25 | 50 | 50 | 50 |
| Cross-linked sodium carboxymethylcellulose (Aqualon R) | - | - | - | - |
| Starch-acrylonitrile graft copolymer (Grain Processing Corp. Polymer 35-A-100) | - | - | - | - |
| Cross-linked dextran (Sephadex CM-C50) | - | - | - | - |
| Poly(methyl vinyl ether/maleic acid), mixed calcium, sodium salt (Gantrez AT-955) | - | - | - | - |

| Ingredient | Example | | | |
|---|---|---|---|---|
| | 16 | 17 | 18 | 19 |
| Guar gum | 20 | - | - | - |
| Locust bean gum | - | - | 20 | - |
| Pectin | 20 | 23.3 | - | 20 |
| Karaya | - | - | 20 | - |
| Gelatin | - | 23.3 | - | 20 |
| Sodium carboxymethylcellulose | 40 | 23.4 | 60 | 20 |
| Calcium carboxymethylcellulose | 20 | - | - | - |
| Collagen | - | - | - | 20 |
| Cross-linked sodium carboxymethylcellulose (Aqualon R) | - | 20 | - | - |
| Starch-acrylonitrile graft copolymer (Grain Processing Corp. Polymer 35-A-100) | - | - | - | 20 |
| Cross-linked dextran (Sephadex CM-C50) | - | - | - | - |
| Poly(methyl vinyl ether/maleic acid), mixed calcium, sodium salt (Gantrez AT-955) | - | 10 | - | - |

| Ingredient | Example | | |
|---|---|---|---|
| | 20 | 21 | 22 |
| Guar gum | - | - | - |
| Locust bean gum | - | - | - |
| Pectin | 20 | 25 | 20 |
| Karaya | - | - | - |
| Gelatin | 20 | 25 | 20 |
| Sodium carboxymethylcellulose | 20 | 25 | 20 |
| Calcium carboxymethylcellulose | - | - | - |
| Collagen | 10 | - | 20 |
| Cross-linked sodium carboxymethylcellulose (Aqualon R) | - | 25 | 20 |
| Starch-acrylonitrile graft copolymer (Grain Processing Corp. Polymer 35-A-100) | - | - | - |
| Cross-linked dextran (Sephadex CM-C50) | 20 | - | - |
| Poly(methyl vinyl ether/maleic acid), mixed calcium, sodium salt (Gantrez AT-955) | 10 | - | - |

The use of the occlusive dressings described above results in a closed moist wound treatment environment. Unlike gauze type dressings, the ingredients employed in adhesive layer 14 permit the dressings of this invention to remain in place over the wound for up to several days. It is believed that the need to frequently change a dressing disturbs the wound healing environment and results in a slower healing process.

The water dispersable hydrocolloid materials, distributed throughout adhesive layer 14 react in the presence of moisture. In the area of normal skin surrounding the wound site, the layer 14 will gradually hydrate over a period of days. The initial aggressive bond of the dressing to this normal skin is due to the presence of the pressure sensitive adhesive materials in layer 14; i.e., dry tack. As this bond is lessened by perspiration and leakage of moisture under layer 14, the wet tack of the moisture active ingredients in layer 14 becomes more critical in bonding the dressing to the skin. Eventually, layer 14 becomes so hydrated that the dressing can be removed without stripping or macerating the skin around the wound site.

When the dressing is applied over a fluid emitting wound such as a dermal ulcer, it has been observed that the moisture active ingredients in layer 14 will be converted to an almost gel-like mass. This provides an ideal moist environment for cell migration ensuring easy dressing removal with a minimum of damage to the newly formed tissues.

In treating ulcers emitting a large volume of exudate, it has been found to be useful to first pack the wound with the granular material described above and then cover with the occlusive dressing. The granules interact with the wound exudate to form a gel-like mass and prevent leakage through the dressing. As a result, the dressing is changed less often which is believed to result in a shortened healing period. After removal of the dressing, the hydrated granules can be removed from the wound site by flushing with saline solution. As healing progresses and less exudate is present in the sound site, the dressing can be employed without the granular packing.

Fig. 7 shows an ulcer 70 covered by occlusive dressing 10. The dressing is partially broken away to show the granular packing material 75. of course, adhesive layer 14 contacts the granules 75 in the area of the wound and also bonds the dressing to the normal skin 71 surrounding the ulcer.

**Claims**

1. An occlusive multi-layered dressing (10, 30, 50) comprising an adhesive layer (14) which, in use, contacts the wound and surrounding normal skin, an intermediate layer (12) of semi-open-cell polymeric foam, and an outer moisture impervious polymeric film (11) coated or laminated to the upper surface of said foam layer (12), wherein said wound and skin contacting adhesive layer (14) consists essentially of a homogeneous blend of from about 35% to about 50% by weight of one or more low molecular weight polyisobutylenes which act as pressure sensitive adhesive materials and from about 45% to about 65% by weight of one or more water dispersable hydrocolloids selected from sodium carboxymethylcellulose, calcium carboxymethylcellulose, pectin, gelatin, guar gum, locust bean gum, collagen, and gum karaya.

2. The dressing of claim 1 wherein said wound and skin contacting adhesive layer (14) is a homogeneous blend of about 40% by weight of low molecular weight polyisobutylene, about 20% by weight of sodium carboxymethylcellulose, about 20% by weight of pectin, and about 20% by weight of gelatin.

3. The dressing of claim 2 wherein said open-cell polymeric foam (12) is polyurethane foam and said water-impervious polymeric film (11) is polyurethane.

4. The dressing of claim 3 wherein said wound and skin contacting adhesive layer (14) is from about 0.02 inches (0.5 mm) to about 0.1 inches (2.54 mm) in thickness, said polyurethane foam layer (12) is about 0.03 inches (0.76 mm) to about 0.1 inches (2.54 mm) in thickness, and said water-impervious polyurethane layer (11) is about 0.001 inches (0.0254 mm) to about 0.003 inches (0.076 mm) in thickness.

5. The dressing of any preceding claim wherein said wound and skin contacting adhesive layer (14) is bonded directly to the bottom surface of said foam layer (12).

6. The dressing of claim 1 wherein said wound and skin contacting adhesive layer (14) is bonded to said open-cell polymeric foam (12) by a second adhesive layer (13) and said second adhesive layer (13) consists essentially of a homogeneous blend of from about 30% to about 70% by weight of one or more pressure sensitive adhesive materials and one or more optional thermoplastic elastomers, from about 10% to about 65% by weight of one or more water dispersable hydrocolloids and up to 50% by weight of one or more optional water swellable cohesive strengthening agents and/or one or more optional hydratable polymers provided that said water dispersable hydrocolloids, water swellable cohesive strengthening agents, and hydratable polymers together are present at no more than about 70% by weight of said adhesive layer, from about 5% to about 15% by weight of a plasticizer or solvent, and from about 15% to about 25% by weight of a tackifier.

7. The dressing of claim 6 wherein, in said second adhesive layer (13), said pressure sensitive adhesive material is selected from natural rubber, silicone rubber, acrylonitrile rubber, polyurethane rubber, and low molecular weight polyisobutylenes, said optional thermoplastic elastomer if present is selected from medium molecular weight polyisobutylenes, butyl rubber, styrene-butadiene-styrene copolymers, styrene-isoprene-styrene copolymers, and styrene-ethylene/butylenestyrene copolymers, said water dispersable hydrocolloid is selected from sodium carboxymethylcellulose, calcium carboxymethylcellulose, pectin, gelatin, guar gum, locust bean gum, collagen, and gum karaya, said optional water swellable cohesive strengthening agent if present is selected from water-insoluble cross-linked sodium carboxymethylcellulose, water-insoluble starch-acrylonitrile graft copolymer, and water-insoluble cross-linked dextran, said optional hydratable polymer if present is selected from gluten and long chain polymers of methyl vinyl ether/maleic acid, said plasticizer or solvent is mineral oil, and said tackifier is a terpene resin.

8. The dressing of claim 7 wherein said second adhesive layer (13) is a homogeneous blend of from about 35% to about 50% by weight of low molecular weight polyisobutylenes and one or more optional thermoplastic elastomers selected from medium molecular weight polyisobutylenes, butyl rubber, and styrene-isoprene-styrene copolymers, from about 30% to about 50% by weight of one or more water dispersable hydrocolloids, one or more optional water swellable cohesive strengthening agents, and one or more optional hydratable polymers, from about 5% to about 15% by weight of mineral oil and from about 15% to about 25% by weight of a terpene resin tackifier.

9. The dressing of claim 8 wherein said wound and skin contacting adhesive layer (14) is a homogeneous blend of about 40% by weight of low molecular weight polyisobutylene, about 20% by weight of sodium carboxymethylcellulose, about 20% by weight of pectin, and about 20% by weight of gelatin.

10. The dressing of claim 9 wherein said second adhesive layer (13) is a homogeneous blend of from about 18% by weight of low molecular weight polyisobutylenes, about 20% by weight of medium molecular weight polyisobutylenes, about 18% by weight of sodium carboxymethylcellulose, about 15% by weight of gelatin, about 20% by weight of terpene resin, about 8.5% by weight of mineral oil, and about 0.5% by weight of butylated hydroxytoluene.

11. The dressing of claim 10 wherein said open-cell polymeric foam is polyurethane foam and said water impervious polymeric film is polyurethane.

12. The dressing of claim 11 wherein said wound and skin contacting adhesive layer (14) is from about 0.02 inches (0.5 mm) to about 0.1 inches (2.54 mm) in thickness, said second adhesive layer is from about 0.005 inches (0.127 mm) to about 0.02 inches (0.5 mm) in thickness, said polyurethane foam layer is about 0.03 inches (0.76 mm) to about 0.1 inches (2.54 mm) in thickness, and said water-impervious polyurethane layer is about 0.001 inches (0.0254 mm) to about 0.003 inches (0.076 mm) in thickness.

13. The dressing of claim 6 wherein a layer (31) of deodorizing material is interposed between the two adhesive layers (14, 13).

14. The dressing of claim 13 wherein said layer (31) of deodorizing material contains activated carbon.

15. A method for preparing a multi-layered dressing according to claim 6 which comprises forming a homogeneous dough, consisting essentially of the one or more low molecular weight polyisobutylenes and the one or more hydrocolloids, by mechanical mixing, forming an adhesive layer by mixing one or more hydrocolloid gums, one or more pressure sensitive adhesive materials, tackifier and plasticizer in an organic solvent such as heptane or hexane, applying the resulting adhesive slurry to a web of silicone coated release paper and evaporating the solvent so dispersing the dried hydrocolloid throughout the adhesive layer, compressing with a laminate of semi-open-cell flexible polymeric foam having a water-impermeable polymeric coating or skin on one side, and stripping away the silicone coated release paper from the adhesive layer and pressing together with heat the adhesive layers to form the dressing.

**Revendications**

1. Pansement multicouche occlusif (10, 30, 50) comprenant une couche d'adhésif (14) qui, à l'utilisation, est en contact avec la plaie et la peau normale qui l'entoure, une couche intermédiaire (12) de mousse polymère à cellules semi-ouvertes, et une pellicule polymère externe (11) imperméable à l'humidité, étalée ou plaquée sur la surface supérieure de ladite couche de mousse (12), dans lequel ladite couche d'adhésif (14) qui est en contact avec la plaie et la peau se compose essentiellement d'un mélange homogène d'environ 35% à environ 50% en poids d'un ou plusieurs polyisobutylènes à bas poids moléculaire qui jouent le rôle de matières adhésives sensibles à la pression, et d'environ 45% à environ 65% en poids d'un ou plusieurs hydrocolloïdes dispersibles dans l'eau, choisis entre la carboxyméthylcellulose sodique, la carboxyméthylcellulose calcique, la pectine, la gélatine, le guar, la gomme tragasol, le collagène et le karaya.

2. Pansement selon la revendication 1, dans lequel ladite couche d'adhésif (14) en contact avec la plaie et la peau est un mélange homogène d'environ 40% en poids de polyisobutylène de bas poids moléculaire, d'environ 20% en poids de carboxyméthylcellulose sodique, d'environ 20% en poids de pectine, et d'environ 20% en poids de gélatine.

3. Pansement selon la revendication 2, dans lequel ladite mousse polymère à cellules semi-ouvertes (12) est une mousse de polyuréthane et ladite pellicule polymère imperméable à l'eau (11) est une pellicule de polyuréthane.

4. Pansement selon la revendication 3, dans lequel ladite couche d'adhésif (14) en contact avec la plaie et la peau a d'environ 0,02 pouce (0,5 mm) à environ 0,1 pouce (2,54 mm) d'épaisseur, ladite couche de mousse de polyuréthane (12) a d'environ 0,03 pouce (0,76 mm) à environ 0,1 pouce (2,54 mm) d'épaisseur, et ladite couche de polyuréthane imperméable à l'eau (11) a d'environ 0,001 pouce (0,0254 mm) à environ 0,003 pouce (0,076 mm) d'épaisseur.

5. Pansement selon l'une quelconque des revendications précédentes, dans lequel ladite couche d'adhésif (14) en contact avec la plaie et la peau est liée directement à la surface inférieure de ladite couche de mousse (12).

6. Pansement selon la revendication 1, dans lequel ladite couche d'adhésif (14) en contact avec la plaie et la peau est liée à ladite mousse polymère à cellules semi-ouvertes (12) par une seconde couche d'adhésif (13), et ladite seconde couche d'adhésif (13) se compose essentiellement d'un mélange homogène d'environ 30% à environ 70% en poids d'une ou plusieurs matières adhésives sensibles à la pression et d'un ou plusieurs élastomères thermoplastiques facultatifs, d'environ 10% à environ 65% en poids d'un ou plusieurs hydrocolloïdes dispersibles dans l'eau, et jusqu'à 50% en poids d'un ou plusieurs agents facultatifs de renforcement de la cohésion gonflant dans l'eau et/ou d'un ou plusieurs polymères hydratables facultatifs, à condition que lesdits hydrocolloïdes dispersibles dans l'eau, lesdits agents de renforcement de la cohésion gonflant dans l'eau et lesdits polymères hydratables ne représentent ensemble pas plus d'environ 70% du poids de ladite couche d'adhésif, d'environ 5% à environ 15% en poids d'un plastifiant ou d'un solvant, et d'environ 15% à environ 25% en poids d'un agent d'adhérence.

7. Pansement selon la revendication 6, dans lequel ladite matière sensible à la pression de ladite seconde couche d'adhésif (13) est choisie entre le caoutchouc naturel, le caoutchouc de silicone, le caoutchouc d'acrylonitrile, le caoutchouc de polyuréthane et les polyisobutylènes de bas poids moléculaire, ledit élastomère thermoplastique facultatif, s'il est présent, est choisi entre les polyisobutylènes à poids moléculaire moyen, le caoutchouc butyl, les copolymères de styrène-butadiène-styrène, les copolymères de styrène-isoprène-styrène, et les copolymères de styrène-éthylène/butylène-styrène, ledit hydrocolloïde dispersible dans l'eau est choisi entre la carboxyméthylcellulose sodique, la carboxyméthylcellulose calcique, la pectine, la gélatine, le guar, la gomme tragasol, le collagène et le karaya, ledit agent facultatif de renforcement de la cohésion gonflant dans l'eau, s'il est présent, est choisi entre la carboxyméthylcellulose sodique réticulée insoluble dans l'eau, un copolymère greffé d'amidon et d'acrylonitrile insoluble dans l'eau, et un dextranne réticulé insoluble dans l'eau, ledit polymère hydratable facultatif, s'il est présent, est choisi entre le gluten et les polymères à longue chaîne d'éther méthyl vinylique et d'acide maléique, ledit plastifiant ou solvant est une huile minérale, et ledit agent d'adhérence est une résine terpénique.

8. Pansement selon la revendication 7, dans lequel ladite seconde couche d'adhésif (13) est un mélange homogène d'environ 35% à environ 50% en poids de polyisobutylènes à bas poids moléculaire et d'un ou plusieurs élastomères thermoplastiques facultatifs choisis entre les polyisobutylènes à poids moléculaire moyen, le caoutchouc butyl, et les copolymères de styrène-isoprène-styrène, d'environ 30% à environ 50% en poids d'un ou plusieurs hydrocolloïdes dispersibles dans l'eau, d'un ou plusieurs agents facultatifs de renforcement de la cohésion gonflant dans l'eau, et d'un ou plusieurs polymères hydratables facultatifs, d'environ 5% à environ 15% d'huile minérale, et d'environ 15% à environ 25% en poids d'un agent d'adhérence qui est une résine terpénique.

9. Pansement selon la revendication 8, dans lequel ladite couche d'adhésif (14) en contact avec la plaie et la peau est un mélange homogène d'environ 40% en poids de polyisobutylène à bas poids moléculaire, d'environ 20% en poids de carboxyméthylcellulose sodique, d'environ 20% en poids de pectine, et d'environ 20% en poids de gélatine.

10. Pansement selon la revendication 9, dans lequel ladite seconde couche d'adhésif (13) est un mélange homogène d'environ 18% en poids de polyisobutylènes à bas poids moléculaire, d'environ 20% en poids de polyisobutylènes à poids moléculaire moyen, d'environ 18% en poids de carboxyméthylcellulose sodique, d'environ 15% en poids de gélatine, d'environ 20% en poids de résine terpénique, d'environ 8,5% en poids d'huile minérale, et d'environ 0,5% en poids d'hydroxytoluène butylé.

11. Pansement selon la revendication 10, dans lequel ladite mousse polymère à cellules semi-ouvertes est une mousse de polyuréthane, et ladite pellicule polymère imperméable à l'eau est une pellicule de polyuréthane.

12. Pansement selon la revendication 11, dans lequel ladite couche d'adhésif (14) en contact avec la plaie et la peau a d'environ 0,02 pouce (0,5 mm) à environ 0,1 pouce (2,54 mm) d'épaisseur, ladite seconde couche d'adhésif a d'environ 0,005 pouce (0,127 mm) à environ 0,02 pouce (0,5 mm) d'épaisseur, ladite couche de mousse de polyuréthane a d'environ 0,03 pouce (0,76 mm) à environ 0,1 pouce (2,54 mm) d'épaisseur, et ladite couche de polyuréthane imperméable à l'eau a d'environ 0,001 pouce (0,0254 mm) à environ 0,003 pouce (0,076 mm) d'épaisseur.

13. Pansement selon la revendication 6, dans lequel une couche (31) de matière désodorisante est interposée entre les deux couches d'adhésif (14, 13).

14. Pansement selon la revendication 13, dans lequel ladite couche (31) de matière désodorisante contient du charbon actif.

15. Procédé de préparation d'un pansement multicouche selon la revendication 6, qui consiste à former, par mélangeage mécanique, une pâte homogène, composée essentiellement du ou des polyisobutylènes à bas poids moléculaire et du ou des hydrocolloïdes, à former une couche d'adhésif en mélangeant une ou plusieurs gommes hydrocolloïdales, une ou plusieurs matières adhésives sensibles à la pression, un agent d'adhérence et un plastifiant dans un solvant organique tel que l'heptane ou l'hexane, à appliquer la dispersion d'adhésif résultante sur une bande de papier anti-adhésif revêtu de silicone, et à faire évaporer le solvant de manière à disperser l'hydrocolloïde séché au sein de la couche d'adhésif, à comprimer avec un stratifié de mousse polymère souple à cellules semi-ouvertes portant sur l'une de ses faces une peau ou un revêtement polymère imperméable à l'eau, et à décoller le papier anti-adhésif revêtu de silicone de la couche d'adhésif, et à presser les couches d'adhésif l'une contre l'autre, en appliquant de la chaleur, pour former le pansement.

**Patentansprüche**

1. Okklusiver mehrschichtiger Wundverband (10, 30, 50), umfassend eine Klebemittelschicht (14), die bei Gebrauch mit der Wunde und der umgebenden normalen Haut in Berührung kommt, eine Zwischenschicht (12) aus polymerem Schaum mit halboffenen Zellen und eine äußere feuchtigkeitsundurchlässige Polymerfolie (11), die auf die obere Fläche der Schaumschicht (12) aufgebracht oder laminiert ist, wobei die Wunde und Haut berührende Klebemittelschicht (14) im wesentlichen aus einem homogenen Gemisch von etwa 35 bis etwa 50 Gew.-% eines oder mehrerer Polyisobutylene niederen Molekulargewichts, die als Haftkleber wirken und aus etwa 45 bis etwa 65 Gew.-% eines oder mehrerer in Wasser dispergierbarer Hydrokolloide, ausgewählt aus Natriumcarboxymethylcellulose, Calciumcarboxymethylcellulose, Pektin, Gelatine, Guargummi, Karobbegummi, Kollagen und Karayagummi besteht.

2. Verband nach Anspruch 1, wobei die Wunde und Haut berührende Klebemittelschicht (14) ein homogenes Gemisch aus etwa 40 Gew.-% eines Polyisobutylens niederen Molekulargewichts, etwa 20 Gew.-% Natriumcarboxymethylcellulose, etwa 20 Gew.-% Pektin und etwa 20 Gew.-% Gelatine ist.

3. Verband nach Anspruch 2, wobei der polymere Schaum mit offenen Zellen (12) ein Polyurethanschaum ist und die wasserundurchlässige Polymerfolie (11) aus Polyurethan besteht.

4. Verband nach Anspruch 3, wobei die Wunde und Haut berührende Klebemittelschicht (14) etwa 0,02 Zoll (0,5 mm) bis etwa 0,1 Zoll (2,54 mm) stark ist, die Polyurethanschaumschicht (12) etwa 0,03 Zoll (0,76 mm) bis etwa 0,1 Zoll (2,54 mm) stark ist und die wasserundurchlässige Polyurethanfolie (11) etwa 0,001 Zoll (0,0254 mm) bis etwa 0,003 Zoll (0,076 mm) stark ist.

5. Verband nach einem vorangehenden Anspruch, wobei die Wunde und Haut berührende Klebemittelschicht (14) direkt an die untere Fläche der Schaumschicht (12) gebunden ist.

6. Verband nach Anspruch 1, wobei die Wunde und Haut berührende Klebemittelschicht (14) durch eine zweite Klebemittelschicht (13) an den polymeren Schaum mit offenen Zellen (12) gebunden ist und die zweite Klebemittelschicht (13) im wesentlichen aus einem homogenen Gemisch aus etwa 30 bis etwa 70 Gew.-% eines oder mehrerer Haftkleber und einem oder mehreren gegebenenfalls thermoplastischen Elastomeren, etwa 10 bis etwa 65 Gew.-% eines oder mehrerer wasserdispergierbarer Hydrokolloide und bis zu 50 Gew.-% eines oder mehrerer gegebenenfalls in Wasser quellbarer kohäsiv verfestigender Mittel und/oder eines oder mehrerer gegebenenfalls hydratisierbarer Polymere; etwa 5 bis etwa 15 Gew.-% eines Weichmachers oder Lösungsmittels und etwa 15 bis etwa 25 Gew.-% eines Haftmittels besteht, mit der Maßgabe, daß die in Wasser dispergierbaren Hydrokolloide, in Wasser quellbaren kohäsiv verfestigenden Mittel und die hydratisierbaren Polymeren zusammen nicht mehr als 70 Gew.-% der Klebemittelschicht ausmachen.

7. Verband nach Anspruch 6, wobei in der zweiten Klebemittelschicht (13) der Haftkleber ausgewählt ist aus Naturkautschuk, Silikonkautschuk, Acrylnitrilkautschuk, Polyurethankautschuk und Polyisobutylenen niederen Molekulargewichts, das gegebenenfalls thermoplastische Elastomer, falls vorhanden, ausgewählt ist aus Polyisobutylenen mittleren Molekulargewichts, Butylkautschuk, Styrol-Butadien-Styrol-Copolymerisaten, Styrol-Isopren-Styrol-Copolymerisaten und Styrol-Ethylen/Butylen-Styrol-Copolymerisaten, das in Wasser dispergierbare Hydrokolloid ausgewählt ist aus Natriumcarboxymethylcellulose, Calciumcarboxymethylcellulose, Pektin, Gelatine, Guargummi, Karobbegummi, Kollagen und Karayagummi, das gegebenenfalls in Wasser quellbare kohäsiv verfestigende Mittel, falls vorhanden, ausgewählt ist aus wasserunlöslicher vernetzter Natriumcarboxymethylcellulose, wasserunlöslichem Stärke-Acrylnitril-Pfropfcopolymerisat und wasserunlöslichem vernetztem Dextran, das gegebenenfalls hydratisierbare Polymer, falls vorhanden, ausgewählt ist aus Gluten und langkettigen Polymerisaten von Methylvinylether/Maleinsäure, der Weichmacher oder das Lösungsmittel Mineralöl ist und das Haftmittel ein Terpenharz ist.

8. Verband nach Anspruch 7, wobei die zweite Klebemittelschicht (13) ein homogenes Gemisch aus etwa 35 bis etwa 50 Gew.-% Polyisobutylenen niederen Molekulargewichts und einem oder mehreren, gegebenenfalls thermoplastischen Elastomeren, ausgewählt aus Polyisobutylenen mittleren Molekulargewichts, Butylkautschuk und Styrol-Isopren-Styrol-Copolymerisaten, etwa 30 bis 50 Gew.-% eines oder mehrerer wasserdispergierbarer Hydrokolloide, einem oder mehreren gegebenenfalls in Wasser quellbaren kohäsiv verfestigenden Mitteln und einem oder mehreren gegebenenfalls hydratisierbaren Polymeren, etwa 5 bis etwa 15 Gew.-% Mineralöl und etwa 15 bis etwa 25 Gew.-% eines Terpenharz-Haftmittels ist.

9. Verband nach Anspruch 8, wobei die Wunde und Haut berührende Klebemittelschicht (14) ein homogenes Gemisch aus etwa 40 Gew.-% Polyisobutylen niederen Molekulargewichts, etwa 20 Gew.-% Natriumcarboxymethylcellulose, etwa 20 Gew.-% Pektin und etwa 20 Gew.-% Gelatine ist.

10. Verband nach Anspruch 9, wobei die zweite Klebemittelschicht (13) ein homogenes Gemisch aus etwa 18 Gew.-% Polyisobutylenen niederen Molekulargewichts, etwa 20 Gew.-% Polyisobutylenen mittleren Molekulargewichts, etwa 18 Gew.-% Natriumcarboxymethylcellulose, etwa 15 Gew.-% Gelatine, etwa 20 Gew.-% Terpenharz, etwa 8,5 Gew.-% Mineralöl und etwa 0,5 Gew.-% butyliertes Hydroxytoluol ist.

11. Verband nach Anspruch 10, wobei der polymere Schaum mit offenen Zellen ein Polyurethanschaum ist und die wasserundurchlässige Polymerfolie aus Polyurethan besteht.

12. Verband nach Anspruch 11, wobei die Wunde und Haut berührende Klebemittelschicht (14) etwa 0,02 Zoll (0,05 mm) bis etwa 0,1 Zoll (2,54 mm) stark ist, die zweite Klebemittelschicht etwa 0,005 Zoll (0,127 mm) bis etwa 0,02 Zoll (0,5 mm) stark ist, die Polyurethanschaumschicht etwa 0,03 Zoll (0,76 mm) bis etwa 0,1 Zoll (2,54 mm) stark ist und die wasserundurchlässige Polyurethanschicht etwa 0,001 Zoll (0,0254 mm) bis etwa 0,003 Zoll (0,076 mm) stark ist.

13. Verband nach Anspruch 6, wobei eine Schicht (31) aus desodorierendem Material zwischen den zwei Klebemittelschichten (14, 13) eingefügt ist.

14. Verband nach Anspruch 13, wobei die Schicht (31) des desodorierenden Materials Aktivkohle enthält.

15. Verfahren zur Herstellung eines Mehrschichtverbands gemäß Anspruch 6, umfassend die Herstellung eines homogenen Teiges, im wesentlichen bestehend aus einem oder mehreren Polyisobutylenen niederen Molekulargewichts und einem oder mehreren Hydrokolloiden, durch mechanisches Vermischen, Herstellen einer Klebeschicht durch Vermischen eines oder mehrerer Hydrokolloidgummis, eines oder mehrerer Haftkleber, Haftmittel und Weichmacher in einem organischen Lösungsmittel, wie Heptan oder Hexan, Aufbringen der so erhaltenen Klebemittelaufschlämmung auf ein silikonbeschichtetes Abstreifpapier und Verdampfen des Lösungsmittels wodurch das getrocknete Hydrokolloid in der gesamten Klebemittelschicht dispergiert wird, Aufdrücken eines Laminats aus flexiblem polymeren Schaum mit halboffenen Zellen mit einer wasserundurchlässigen polymeren Beschichtung oder Haut auf einer Seite und Abstreifen des silikonbeschichteten Abstreifpapiers von der Klebeschicht und Zusammenpressen der Klebeschichten unter Anwendung von Wärme zum Verband.

Fig. 1

Fig. 2

Fig. 3

Fig. 4

Fig. 5

Fig. 6

Fig. 7